# EUROPEAN PATENT SPECIFICATION

(11) **EP 2 918 677 B1**
(45) Date of publication and mention of the grant of the patent: **09.12.2020**
(21) Application number: 13852198.4
(22) Date of filing: 30.10.2013
(51) Int. Cl.: C12P 19/02, C12P 19/24, C12N 9/90, C12N 15/09

(54) **METHOD FOR PRODUCING D-ALLOSE**
VERFAHREN ZUR HERSTELLUNG VON D-ALLOSE
PROCÉDÉ DE PRODUCTION DE D-ALLOSE

(30) Priority: 30.10.2012 JP 2012239181
(43) Date of publication of application: 16.09.2015
(73) Proprietor: Matsutani Chemical Industry Co., Ltd., Itami-shi, Hyogo 664-8508 (JP); Nagase & Co., Ltd., Osaka 550-8668 (JP); Nagase ChemteX Corporation, Osaka-shi, Osaka 550-8668 (JP)
(72) Inventor: GULLAPALLI, Pushpa Kiran, Itami-shi Hyogo 664-8508 (JP); SHINTANI, Tomoya, Itami-shi Hyogo 664-8508 (JP); NISHIMOTO, Yukifumi, Tokyo 103-8355 (JP); HARAZONO, Koichi, Fukuchiyama-shi Kyoto 620-0853 (JP)
(74) Representative: TBK
(86) International application number: PCT/JP2013/079450
(87) International publication number: WO 2014/069537

(56) References cited:
- EP-A1- 1 589 102
- EP-A1- 1 788 089
- WO-A1-2006/022239
- JP-A- 2002 017 392
- JP-A- 2008 109 933
- CHANG-SU PARK ET AL: "Characterization of a recombinant thermostable-rhamnose isomerase fromATCC 43589 and its application in the production of-lyxose and-mannose", BIOTECHNOLOGY LETTERS, SPRINGER NETHERLANDS, DORDRECHT, vol. 32, no. 12, 1 September 2010 (2010-09-01), pages 1947-1953, XP019859067, ISSN: 1573-6776, DOI: 10.1007/S10529-010-0385-7
- DATABASE UNIPROT/TREMBL [Online] 01 November 1999 'Definition: Streptomyces coelicolor, rhamnose isomerase', XP055265462 Database accession no. Q9XAB3
- DATABASE UNIPROT/TREMBL [Online] 21 May 2012 'Definition: Streptomyces coelicoflavus, rhamnose isomerase', XP055265471 Database accession no. H1QB75
- DATABASE UNIPROT/TREMBL [Online] 05 October 2010 'Definition: Streptomyces griseoflavus, rhamnose isomerase', XP055265482 Database accession no. D9Y207
- DATABASE UNIPROT/TREMBL [Online] 01 June 2003 'Definition: Streptomyces avermitilis, rhamnose isomerase', XP055265486 Database accession no. Q825P2
- YU-RI LIM ET AL.: 'Microbial metabolism and biotechnological production of D-allose' APPL. MICROBIOL. BIOTECHNOL. vol. 91, no. 2, 08 June 2011, pages 229 - 235, XP019920942

## Description

### Technical Field

The present invention relates to novel use of proteins originating in microorganisms of genus Streptomyces and comprising the amino acid sequence represented by SEQ ID NO:1 or SEQ ID NO: 2, specifically to use of enzymes that isomerize D-psicose to D-allose.

The present invention elucidates a sugar isomerization reaction catalyzing function that was found in the enzymes produced by microorganisms of genus Streptomyces and also relates to a technique for producing D-allose by utilizing the novel function.

### Background Art

Simple sugars are broadly classified into aldoses (sugars with an aldehyde group representing the carbonyl group), ketoses (sugars with a ketone group representing the carbonyl group), and sugar alcohols (also known as polyols; sugars without a carbonyl group) according to the state of the reducing group (carbonyl group). Simple sugars include what is known as "rare sugar." International Society of Rare Sugars defines rare sugar as a sugar that occurs rarely in nature, and many of rare sugars are produced in only small yields even by organic chemical syntheses. For this reason, there have been ongoing developments of processes for efficiently producing aldohexose (aldose) rare sugars, including allose, and these rare sugars are being investigated for their unique properties .

Rare sugars are known to have physiological activities. For example, an anti-tumor agent containing a derivative of D-allose as an active ingredient is disclosed (Patent Literature 1). Also disclosed as a technique that takes advantage of sugar properties against reactive oxygen species is, for example, a reactive oxygen production inhibitor containing polysaccharides that have the property to inhibit reactive oxygen species (Patent Literature 2).

Psicose is a hexose with a reducing ketone group. This rare sugar has become relatively easily available after the advent of epimerase. It has been indicated that D-psicose is potentially effective as a raw material or an intermediate of products such as sweeteners, fermentation carbon sources, reagents, cosmetics, and drugs. For example, a hydantoin derivative synthesis using D-psicose as a raw material has been reported as an application of psicose used as an intermediate raw material of products such as reagents and drugs (Non Patent Literature 1).

D-allose can be produced from D-psicose. For example, patent literatures directed to producing D-allose with an enzyme isomerase capable of producing D-allose from D-psicose have been proposed, including, for example, a method for mass production of a pure rare sugar through continuous chromatographic separation of a simulated moving bed as a target rare sugar fraction from a liquid concentrate obtained by the conversion of a substrate rare sugar into a target rare sugar under the catalytic effect of an isomerization catalyzing enzyme (Patent Literature 3), and a D-allose producing method that produces D-allose through isomerization under the effect of a Pseudomonas stutzeri (IPOD FERM BP-08593) -derived protein having an L-rhamnose isomerase activity (Patent Literature 4) .

There is also proposed an aldohexose producing method that produces D-allose and D-altrose from D-psicose, and L-altrose from L-psicose in a D-psicose- and/or L-psicose-containing solution acted upon by D-xylose isomerase, and collects one or more aldohexoses selected from these D-allose, D-altrose, and L-altrose (Patent Literature 5).

### Citation List

### Patent Literature

Patent Literature 1: JP-B-59-40400
Patent Literature 2: JP-A-07-285871
Patent Literature 3: JP-A-2006-153591
Patent Literature 4: JP-A-2008-109933
Patent Literature 5: JP-A-2002-17392
Patent Literature 6: WO2007-058086
Patent Literature 7: US Patent 5,620,960 (1999)
Patent Literature 8: JP-A-2009-153516

### Non Patent Literature

Non Patent Literature 1: Tetrahedron, 47, No.12/13, p2113 (1991)
Non Patent Literature 2: J. Bacteriol., 73:410-414 (1956).
Non Patent Literature 3: J. Biosci Bioeng., 96:89-91 (2003).
Non Patent Literature 4: Davis et al., BASIC METHODS IN MOLECULAR BIOLOGY, 1986
Non Patent Literature 5: Methods Carbohydr. Chem., 1:102-104 (1962).
Non Patent Literature 6: Carbohydr. Res., 24:192-197 (1972).
Non Patent Literature 7: J. Ferment Bioeng., 8: 539-541 (1998).
Non Patent Literature 8: J. Mol. Biol., 300:917-933 (2000). EP 1 788 089 A1 describes the sequence of a thermotolerant L-rhamnose isomerase gene.

CHANG-SU PARK ET AL, "Characterization of a recombinant thermostable-rhamnose isomerase from ATCC 43589 and its application in the production of-lyxose and-mannose", BIOTECHNOLOGY LETTERS, SPRINGER NETHERLANDS, DORDRECHT, (20100901), vol. 32, no. 12, doi:10.1007/S10529-010-0385-7, ISSN 1573-6776, pages 1947 - 1953, describes a recombinant thermostable-rhamnose isomerase.

EP 1 589 102 A1 describes a L-rhamnose isomerase derived from *Pseudomonas stutzerii.*

### Summary of Invention

### Technical Problem

Various techniques have been developed to produce D-allose from a variety of microorganisms. However, actual applications for the production of D-allose for food have been difficult because of the safety issue posed by the microorganisms. This has created a demand for the production of rare sugar with an enzyme originating in microorganisms that do not pose a safety problem.

For rare sugar production, for example, it is known that D-psicose can be produced from D-fructose with a D-ketohexose 3-epimerase originating in Pseudomonas cichorii. However, Pseudomonas cichorii, being a plant pathogen, are not necessarily suitable for food applications. Production of D-psicose with microorganisms of genus Rhizobium has been proposed (Patent Literature 6). However, some Rhizobium microorganisms are plant pathogens, and are not desirable for food applications. Further, these techniques are directed to producing D-psicose, not D-allose.

The present invention is intended to overcome the safety issue of the related art, and it is an object of the present invention to obtain and use an isomerase that can catalyze the isomerization of D-psicose to D-allose in a high yield from putatively essentially non-toxic bacterial strains selected from the strains listed in the List of Existing Additives Directory Items, which contains a list of bacterial strains approved for food production. The present invention provides a D-allose producing method that uses such an enzyme.

### Solution to Problem

The present inventors completed the present invention after long studies conducted to find a D-allose producing microorganism that does not involve the safety issue of the related art, specifically an enzyme produced by such a microorganism. Specifically, the present invention is based on the finding of an enzyme having the activity to convert D-psicose to D-allose and obtained from a created library of actinomycetes isolated from soil. Actinomycetes are unlikely to cause problems in food production when used as the source microorganism of the enzyme for existing additives. An isomerase obtained from actinomycetes can thus be used as a very useful means to mass produce D-allose.

Specifically,the method of the present invention uses the following protein.
(1) A protein as defined in claim 1 comprising the amino acid sequence represented by SEQ ID NO: 1 or SEQ ID NO: 2.
(2) The protein according to (1), wherein the protein isomerizes a ketose D-psicose to an aldose D-allose.
(3) The protein defined in (1) or (2) displays the following physical and chemical properties (d) to (f),
   (d) an effective pH of 6.0 to 11.0, and an optimum pH of 9.0,
   (e) an effective temperature of 10 to 80°C, and an optimum temperature of 60°C, and
   (f) reactivity to L-rhamnose, D-xylose, D-ribose, D-allose, D-glucose, and L-arabinose.

The method of the present invention also uses the DNA of (4), the recombinant vector of (5), and the host cell of (6), below.
(4) A DNA of Streptomyces sp. 710 (Deposition Number: NITE BP-01423) or Streptomyces sp.720 (Deposition Number: NITE BP-01424) origin comprising the base sequence of SEQ ID NO: 3 or SEQ ID NO: 4, a complementary sequence thereof, or a sequence with a part of or all of the base sequence or the complementary sequence, and encoding the protein of (1) or (2) . (5) A recombinant vector comprising the DNA of (4).
(6) A host cell comprising an expression system capable of causing expression of the protein of (1), (2), or (3).
(7) The production of the recombinant protein comprises culturing the host cell with the expression system of (6) in a medium, and collecting the recombinant protein of (1), (2), or (3) from the obtained culture.

The present invention lies in the D-allose producing methods of the following (8) to (12).
(8) A method for producing D-allose,
   the method comprising isomerizing D-psicose to D-allose under the activity of the protein of (1).
(9) The method for producing D-allose according to (8), wherein the D-psicose is produced by epimerizing D-fructose.
(10) The method for producing D-allose according to (8), wherein the D-psicose is produced by directing D-glucose to D-fructose through isomerization, and epimerizing the D-fructose.
(11) The method for producing D-allose according to (8), wherein the D-psicose is produced by obtaining D-glucose from an unused resource, directing the D-glucose into D-fructose through isomerization, and epimerizing the D-fructose.
(12) The method for producing D-allose according to any one of (8) to (11), wherein the target product D-allose is a mixture of D-psicose and D-allose.

### Advantageous Effects of Invention

The present invention, specifically the use of an enzyme protein originating in microorganisms of genus Streptomyces makes it possible to recognize and react with the C1 CHO group and the C2 OH group of an aldose, and convert the C1 CHO group to an OH group and the C2 OH group to a CO group, or recognize and react with the C1 OH group and the C2 CO group of a ketose, and convert the C1 OH group to a CHO group and the C2 CO group to an OH group, preferably produce D-allose from D-psicose. The present invention uses an enzyme having the amino acid sequence of SEQ ID NO: 1 or SEQ ID NO: 2 originating in microorganisms of genus Streptomyces. The enzyme recognizes and reacts with the C1 CHO group and the C2 OH group of an aldose, and converts the C1 CHO group to an OH group and the C2 OH group to a CO group, or recognizes and reacts with the C1 OH group and the C2 CO group of a ketose, and converts the C1 OH group to a CHO group and the C2 CO group to an OH group, and preferably isomerizes D-psicose to D-allose. The present invention with the use of the enzyme enables D-allose production.

The present invention enabling use of highly safe bacteria in terms of bacterial culture is a large technological advancement. The present invention uses the enzyme capable of producing D-allose through isomerization of D-psicose, and the established method of production of such enzymes is thus considered to be industrially highly useful not only in sugar production but in related food, cosmetic, and drug industries. The present invention also enables D-allose production via D-psicose from starting materials such as D-glucose, one of the cheapest and the most widely available sugars, and fructose.

### Brief Description of Drawings

FIG. 1 is a diagram representing the recombinant isomerase configuration (restriction enzyme map) of Example 1.
FIG. 2 is a diagram representing the effect of metal ions on enzyme activity.
FIG. 3 is a diagram representing (a) the effect of temperature for optimum enzyme activity, and (b) stability after 1 hour under a maintained temperature.
FIG. 4 is a diagram representing (a) the effect of pH for optimum enzyme activity, and (b) enzyme stability after 24 hours under a maintained temperature.
FIG. 5 is a diagram representing the substrate specificity of the enzymes of the present invention.
FIG. 6 is a diagram representing the result of HPLC analysis before (D-psicose) and after (a mixture of D-psicose, D-altrose, and D-allose) the reaction.

### Description of Embodiments

D-allose represents a very useful rare sugar, and has an important role in inhibition of cancer cell proliferation. Arnold and Silady have reported that D-allose essentially inhibits production of segmented neutrophils, and lowers platelet numbers without causing other harmful clinical effects (Patent Literature 7). There is also a report of D-allose inhibiting reactive oxygen production (Non Patent Literature 3). D-allose also has been investigated in many studies, particularly with regard to its anticancer effect in the recent years. Under these circumstances, the present inventors conducted intensive studies to provide a technique that can be used to safely produce the useful D-allose in large quantities, with the goal of obtaining an enzyme that converts D-psicose into D-allose from bacterial strains that are safe to use in food production.

L-Rhamnose isomerase (L-RhI, E.C: 5.3.1.14) is an enzyme that catalyzes the isomerization reaction of D-psicose into D-allose. This enzyme, in Escherichia coli, is known to catalyze the reaction that reversibly isomerizes L-rhamnose into a corresponding ketose L-rhamnulose (Non Patent Literature 2). However, Escherichia coli are not necessarily safe for food production.

In order to establish a technique that enables easy production of the industrially promising D-allose, the present inventors created a library of isolated actinomycetes from soil, and searched the library for bacteria that produce an isomerase that converts D-psicose into D-allose.

The protein used as the enzyme that produces D-allose from D-psicose in the present invention is a Streptomyces microorganism-derived protein having the amino acid sequence of SEQ ID NO: 1 or SEQ ID NO: 2 with the activity to convert D-psicose into D-allose.

Typically, the enzyme of interest may be extracted from cultured bacteria, and directly used for the reaction. It is, however, preferable to immobilize the enzyme before use. The enzyme having the foregoing activity and to be immobilized may itself not be necessarily required to be purified to a high purity level as may be decided according to the intended use, and may be used as a crude enzyme. Specifically, the crude enzyme may be, for example, the microorganism itself that is capable of producing the enzyme having the activity, or a culture or a partially purified culture of such microorganisms .

The enzyme of interest for the present invention may be used after being immobilized by using an ordinary method, for example, such as a carrier binding method, a crosslinking method, and an entrapment method. These are not limited to the enzyme, and may be obtained by immobilizing the bacteria itself or the crude enzyme on a carrier such as resin. The immobilization can produce an immobilized enzyme that can remain stable for several months, as opposed to about one week without immobilization.

For example, a D-psicose solution containing 50% ethanol may be passed through an immobilized enzyme and/or an immobilized microorganism obtained after immobilizing the enzyme and/or microorganism of interest for the present invention by using a covalent bonding method as an example of the carrier binding method, and the temperature may be controlled, for example, at 42°C for the reaction, and 4°C for the crystallization, to continuously produce D-allose crystals. The filtrate after the crystallization may be passed again through the immobilized enzyme and/or immobilized microorganism without having the ethanol removed or being concentrated to continuously produce D-allose.

This is innovative in the sense that the method allows separation of only the D-allose by simple addition of ethanol to the mixed solution of D-psicose and D-allose. The method also does not require removing the buffer used for the enzyme reaction, and is highly advantageous in that the separation process is much less laborious, and can be performed with improved efficiency. An enzyme reaction that uses 50% D-psicose as the starting material of D-allose production yields the product as a mixed solution of 35% D-psicose and 15% D-allose, and the D-psicose and D-allose in the enzyme reaction product can be quickly separated to obtain D-allose in high purity.

As an example, the enzyme reaction performed in the present invention for the production of D-allose from D-psicose is typically performed under the following conditions. Typically, an aqueous solution of D-psicose is used as a substrate solution, and the substrate concentration is appropriately selected from 1 to 60 w/v%, desirably 10 to 50 w/v%, further desirably 20 to 40 w/v%. The enzyme reaction temperature is selected from a temperature range that does not deactivate the enzyme, for example, 10 to 85°C, desirably 40 to 80°C, further desirably the optimum temperature of 60°C. For the same reason, the enzyme reaction pH is selected from 6.0 to 11.0, more desirably the optimum pH of 9.0. The enzyme activity is selected from a range of 1 unit or more, desirably 50 to 5,000 units per gram of substrate. The reaction time depends on conditions such as the substrate amount, the enzyme amount, the temperature, and the pH. Taking as an example a reaction performed in a batch instead of using the immobilized enzyme, it is desirable for economy that the reaction be performed for about 2 to 100 hours, more desirably about 5 to 50 hours.

The present invention is concerned with a D-allose producing method that produces D-allose through isomerization of D-psicose acted Upon a protein comprising the amino acid sequence of SEQ ID NO: 1 or SEQ ID NO: 2 having L-rhamnose isomerase activity as defined in claim 1.

The method produces D-allose from D-psicose by using an enzyme produced by highly safe strains of actinomycetes and capable of producing D-allose from D-psicose.

The L-rhamnose isomerase activity of the protein is specified by the following physical and chemical properties (d) to (f).

The enzyme activity of the present invention is specified by the following physical and chemical properties (d) to (f).
(d) Effective pH and optimum pH
   The effective pH is 6.0 to 11.0, and the optimum pH is 9.0.
(e) Effective temperature and optimum temperature The effective temperature is 10 to 80°C, and the optimum temperature is 60°C.
(f) Reactivity to L-rhamnose, D-xylose, D-ribose, D-allose, D-glucose, and L-arabinose.

### [D-Psicose]

The D-psicose used in the present invention may be produced by using various methods, including, for example, a method that epimerizes D-fructose, a method that directs D-glucose to D-fructose through isomerization, and epimerizes the D-fructose, and a method that obtains D-glucose from an unused resource, directs the D-glucose to D-fructose through isomerization, and epimerizes the D-fructose.

The present invention is described below in greater detail using Examples. It should be noted, however, that the present invention is in no way limited by the following Examples but defined by the claims.

### Example 1

### [Obtaining Bacterial Strains that Produce Enzyme Capable of D-Psicose to D-Allose Conversion]

Soil (0.1 g) collected from an environment was suspended in 1 mL of sterile distilled water. The suspension was diluted 50 times with sterile distilled water, and 0.1 mL of the diluted suspension was inoculated on an actinomycete isolation medium, and cultured at 30°C for 3 days. After culture, the resulting colonies were picked up (about 200 strains) . The actinomycete medium of the composition presented in Table 1 was used as the actinomycete isolation medium.

**[Table 1]**

| Actinomycete medium | (g/L) |
|---|---|
| Potato starch | 1.0 |
| K₂HPO₄ | 0.03 |
| NaCl | 0.5 |
| MgSO₄·7H₂O | 0.1 |
| NaNO₃ | 0.1 |
| Agar | 20 |

Subsequently, the about 200 bacterial strains thus obtained were each inoculated in 5 mL of the producing medium below in a test tube, and shake cultured at 28°C, 220 rpm shaking conditions. On day 7 of culture, each culture (15 mL) was collected into a centrifuge tube, and centrifuged at 9000 rpm for 10 minutes with a HITACHI High-Speed Micro Centrifuge (Model CF15RXII). After discarding the supernatant, the bacteria were washed once with distilled water, and recentrifuged. After discarding the supernatant, the bacteria were each suspended in 2 mL of a 40 mM tris-maleate buffer (pH 7.0), and sonicated twice, each for 20 seconds, with an Astrason Ultrasonic Cell Disrupter (W385; HEAT SYSTEM) operated in a 50% duty cycle at an output control level of 5. The disrupted solution was centrifuged at 9000 rpm for 10 minutes, and the supernatant was collected to obtain a crude enzyme solution. The crude enzyme solution was adjusted to a protein concentration of 0.10 mg/50 µL, and used as an enzyme sample for the enzyme activity measurement below.

### [Composition of Producing Medium]

A sugar medium (1.0% D-psicose, 0.1% D-tagatose, 0.1% glycerol) and a mineral medium (0.26% ammonium sulfate, 0.24% potassium dihydrogen phosphate, 0.56% dipotassium hydrogen phosphate, 0.01% magnesium sulfate heptahydrate, 0.05% yeast extract; pH 7.0) are each sterilized at 121°C for 20 minutes, and aseptically mixed in equal amounts.

### [Enzyme Activity Checking Methods]

The enzyme samples obtained as above were each checked for isomerase activity by using a HPLC method and/or a cysteine carbazole method.

### HPLC Method:

An enzyme reaction was performed with the composition presented in Table 2. After quenching the enzyme reaction by processing the reaction mixture at 100°C for 2 minutes, the sample was cooled to room temperature, and subjected to a purification process with an ion-exchange resin and a filter to prepare a sample for HPLC. The HPLC sample was fed to a CK08EC column (80°C; Mitsubishi Chemical Corporation), and eluted with purified water at a flow rate of 0.4 mL/min. The generated sugar composition was then measured with a Tosoh Detector RI-8020.

### Cysteine Carbazole Method:

An enzyme reaction was performed with the composition presented in Table 2. After quenching the enzyme reaction by processing the reaction mixture at 100°C for 2 minutes, the sample was cooled to room temperature, and 50 µL of 10% trichloroacetic acid was added to 500 µL of the enzyme reaction solution to quench the reaction. The sample (550 µL) was maintained at 20°C for 1 minute after adding 100 µL of a cysteine solution and 3 mL of 70% sulfuric acid thereto, and heated at 35°C for 20 minutes after adding 100 µL of a carbazole solution thereto. The generated sugar composition was then measured by reading the absorbance at 540 nm.

**[Table 2]**

| Reaction Conditions for Checking Enzyme Activity | |
|---|---|
| Reaction Conditions | |
| Buffer (Gly-NaOH) | 350 µL |
| Substrate (50 mM D-psicose) | 50 µL |
| Enzyme sample (0.10 mg/50 µL) | 50 µL |
| Reaction temperature | 60°C |
| Reaction time | 10 min |

Two strains with the desired high enzyme activity (Streptomyces sp.710 strain, and Streptomyces sp.720 strain; the genus was identified from the 16S rRNA base sequence) were obtained after the foregoing series of procedures. These bacterial strains Streptomyces sp.710 and Streptomyces sp.720 have been internationally deposited at the National Institute of Technology and Evaluation Patent Microorganism Depositary (NITE; 2-5-8 Kazusa Kamatari, Kisarazu, Chiba, Japan) with the deposition numbers NITE BP-01423 and NITE BP-01424, respectively (original deposition date: October 10, 2012), under the Budapest Treaty, and are available therefrom. The 710 and 720 strains originally deposited therein were transferred to the international depositary under the Budapest Treaty upon filing requests on October 7, 2013, and October 30, 2013, respectively, and have been received thereby. The proof of receipt of international depositary for these two strains was issued on October 30, 2013.

### Example 2

Shotgun cloning was performed for the Streptomyces sp.710 and 720 strains.

### [Preparation of Streptomyces sp.710 (or 720) Strain Chromosomes]

Streptomyces sp.710 (or 720) strain was cultured at 30°C for 2 days under 160 rpm in a tryptic soy broth medium (Becton, Dickinson and Company) supplemented with 0.5% glycine (50 mL) . The culture was centrifuged (3,000 rpm, 10 min, 4°C), and the bacteria were collected. After washing the bacteria with TS buffer (10.3% sucrose, 50 mM Tris buffer (pH 8.0), 25 mM EDTA·2Na) (30 mL), the sample was recentrifuged (3,000 rpm, 10 min, 4°C), and the bacteria were collected. The bacteria were then allowed to stand at 37°C for 60 minutes after adding a TS buffer (10 mL) containing 0.5% lysozyme and 0.002% N-acetylmuramidase. Thereafter, a 10% SDS aqueous solution (2.4 mL), and a TS buffer (0.4 mL) containing 2 mg/mL of proteinase K were added to the protoplast-like bacteria solution, and the mixture was slowly stirred, and allowed to stand at 37°C for 60 minutes, and at 50°C for 30 minutes. To the mixture were then added a 5 M sodium chloride aqueous solution (2 mL), and a 0.7 M sodium chloride aqueous solution (1.6 mL) of 10% cetyltrimethylammonium bromide that had been heated to 65°C. The mixture was slowly stirred, and allowed to stand at 65°C for 20 minutes. Thereafter, a chloroform:isoamylalcohol solution (24:1, 20 mL) was added, and the mixture was stirred with a pipette. After centrifugation (12,000 rpm, 10 min, 4°C), isopropyl alcohol was added to the upper layer in 0.6 times the amount of the upper layer, and the resulting precipitate was collected. The precipitate was washed with 70% ethanol, and vacuum dried. After the vacuum drying, the precipitate was dissolved in 10 mL of a TE buffer (10 mM Tris buffer, pH 8.0; 1 mM EDTA·2Na) containing 0.5 mg of Rnase to prepare a chromosome solution of Streptomyces sp. 710 (or 720) strain (preserved at 4°C).

### Example 3

### [Partial Decomposition of Chromosomes]

A 10 × H buffer (Takara Bio; 4.6 µL), and 0.25 units of restriction enzyme Sau3AI were added to the chromosome solution of Streptomyces sp.710 (or 720) strain (40.4 µL), and the mixture was allowed to stand for 40 minutes at 37°C. After adding a 0.5 M EDTA aqueous solution (50 µL), the sample was electrophoresed on a 0.5% low-melting-point agarose gel, and an agarose gel fraction containing DNA fragments of 23 kb or more was cut out. The cut gel block was put in distilled water (50 mL), and was slowly shaken for 15 minutes . After repeating the same procedure, a 10 × β-agarase buffer was added until the final concentration was 1 × with respect to the weight of the gel block, and the gel was allowed to stand at 68°C until it melted. The molten gel was allowed to stand for 10 minutes at 40°C, and β-agarase (3 units per 0.5 g of the gel) was added. The gel was then allowed stand for 60 minutes at 40°C. After adding a 5 M sodium chloride aqueous solution to make the final concentration 0.5 M, the mixture was allowed to stand on ice for 15 minutes. After centrifugation (15,000 rpm, 15 min, 4°C), ethanol was added to the supernatant in 3 times the amount of the supernatant. The mixture was allowed to stand at -80°C for 10 minutes, and centrifuged (15,000 rpm, 15 min, 4°C). Thereafter, 70% ethanol was added to the resulting precipitate, and the mixture was centrifuged (15,000 rpm, 15 min, 4°C). The precipitate was vacuum dried, and dissolved in distilled water (8 µL). The mixture was allowed to stand for 60 minutes at 37°C after adding a 10 × alkaline phosphatase buffer (1 µL) and alkaline phosphatase (1 µL). Thereafter, distilled water (90 µL) and a phenol:chloroform:isoamylalcohol solution (25:24:1, 100 µL) were added, and the mixture was stirred, and centrifuged (15,000 rpm, 10 min, 25°C) . Ethanol was then added to the upper layer in 3 times the amount of the upper layer. The mixture was allowed to stand at -80°C for 10 minutes, and centrifuged (15,000 rpm, 15 min, 4°C). Thereafter, 70% ethanol was added to the resulting precipitate, and the mixture was centrifuged (15,000 rpm, 15 min, 4°C). The precipitate containing the partially decomposed DNA fragments of the Streptomyces sp.710 (or 720) strain chromosomes was vacuum dried, and dissolved in distilled water (5 µL).

### Example 4

### [Creation of Streptomyces sp.710 (or 720) Strain Chromosome Cosmid Library]

Ten micrograms of Actinomycete-Escherichia coli shuttle cosmid vector pTOYAMAcos (Hiroyasu Onaka et al., J. Antibiotics, 2003, Vol. 56, pp. 950-956) was decomposed with restriction enzyme BamHI, and was electrophoresed on an agarose gel. An agarose gel fraction containing a 8.3 kb DNA fragment was then cut out from the gel. The DNA fragment was purified from the cut gel block with a Geneclean Kit (Q-Biogene), and dissolved in distilled water (2.5 µL). To this was added the partially digested DNA fragment aqueous solution (5 µL) of the Streptomyces sp. XP-710 and XP-720 strain chromosomes obtained in the Example, and the mixture was allowed to stand for 17 hours at 16°C after adding a ligation kit (Takara Bio; 7.5 µL). The plasmid in the ligation solution was then introduced into conjugative Escherichia coli by using a Gigapack III Packaging Extract Kit (Stratagene). The Escherichia coli with the plasmid was cultured at 30°C for 2 days in an LB agar medium supplemented with carbenicillin (50 µg/L), and 1,000 colonies collected from the grown transformant (Escherichia coli) were cultured at 30°C for 1 day in a separate LB agar medium supplemented with carbenicillin (50 µg/L).

### Example 5

### [Introduction of Plasmid from Escherichia coli Cosmid Library into Actinomycete]

The 1,000 colonies of Escherichia coli with the cosmid plasmid from Example 4 were each cultured at 30°C in an LB medium (5 mL) supplemented with kanamycin (50 µg/mL) until the absorbance at 660 nm was 0.6. The culture (2 mL) was transferred to a sterilized Eppendorf tube, and centrifuged (3,000 rpm, 5 min, 4°C). After decanting the supernatant, an LB medium (1 mL) was added, and the culture was vigorously agitated to wash the bacteria. The culture was centrifuged (3,000 rpm, 5 min, 4°C), and the supernatant was decanted. This procedure was repeated two more times. After adding an LB medium (0.5 mL), the bacteria were suspended therein. Thereafter, a spore suspension (1 µL) of Streptomyces lividans 1326 strain (NBRC Number: 15675), and a suspension (0.1 mL) of the conjugative Escherichia coli with the plasmid were thoroughly mixed in a separately prepared sterilized Eppendorf tube, and spread over an actinomycete medium Daigo No. 4 (Nihon Pharmaceutical Co., Ltd.). After culturing the cells at 30°C for 18 hours, a nutrient broth (Becton, Dickinson and Company) agar medium (agar 0.5%, 3 mL) supplemented with thiostrepton (167 µg/mL) and nalidixic acid Na (67 µg/mL) was layered, and the culture was continued for 3 days to obtain grown colonies (each colony obtained came from a single Escherichia coli cell).

### Example 6

### [Obtaining Recombinant Actinomycete Capable of Isomerase Production]

The 1,000 colonies produced in Example 5 were each inoculated in 50 mL of the foregoing medium in a 500-mL baffled flask, and rotary cultured under 28°C, 160 rpm conditions. On day 3 of culture, each culture was collected into a 50-mL centrifuge tube. The cells were centrifuged at 9000 rpm for 10 minutes with a HITACHI High-Speed Micro Centrifuge (Model CF15RXII), and the supernatant was discarded. The bacteria were washed once with distilled water, and recentrifuged. After discarding the supernatant, the bacteria were each suspended in 30 ml of a 40 mM tris-maleate buffer (pH 7.0), and sonicated twice, each for 20 seconds, with an Astrason Ultrasonic Cell Disrupter (W385; HEAT SYSTEM) operated in a 50% duty cycle at an output control level of 5. The disrupted solution was centrifuged at 9000 rpm for 10 minutes, and the supernatant was collected. The liquid was then membrane filtered with a Minisart (1.2 µm; Sartorius) to obtain a crude enzyme solution (for 1,000 colonies).

These crude enzyme solutions were checked for activity by using the method described above. The result confirmed activity in the crude enzyme solution of one of the colonies.

An analysis of the DNA sequences of the cosmid library from this colony found a structural gene sequence of uncertain function. An experiment was thus conducted to express this structural gene.

### Example 7

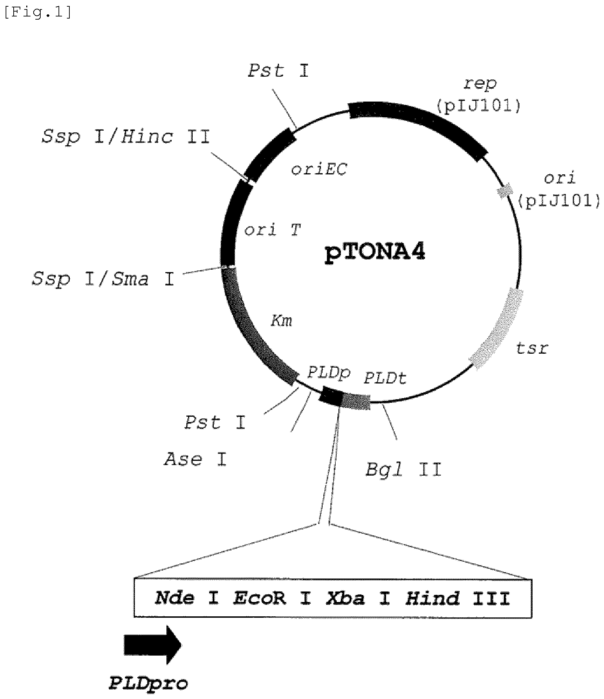

In this Example, a PCR fragment was introduced into a conjugative actinomycete plasmid pTONA4 (Patent Literature 8), and expressed in Streptomyces lividans 1326 strain (NBRC Number: 15675) to obtain the recombinant isomerase configuration (restriction enzyme map) shown in FIG. 1.

Genomic DNA was isolated from each of the Streptomyces sp.710 and 720 stains by using an InstaGene™ Matrix (BIO-RAD) . A forward primer (SEQ ID NO: 5) and a reverse primer (SEQ ID NO: 6) were synthesized. These two primers were used to perform a PCR reaction, using the genomic DNA of the Streptomyces sp. 710 strain as a template. The PCR reaction was performed with a Phusion DNA Polymerase (FINNZYMES) in 35 cycles, each cycle consisting of 98°C for 10 seconds, 58°C for 10 seconds, and 72°C for 2 minutes, in sequence. The resulting PCR fragment of the PCR reaction was processed with restriction enzymes NdeI and HindIII. The restriction enzymes NdeI and HindIII were also used to process the conjugative actinomycete plasmid pTONA4. The obtained two fragments were ligated, and introduced into Streptomyces lividans 1326 strain (NBRC Number: 15675) to obtain an XGP-710 strain. It was confirmed that the resulting PCR fragment was identical to SEQ ID NO: 3 upon determining its base sequence in a DNA sequencer analysis.

Separately, a forward primer (SEQ ID NO: 7) and a reverse primer (SEQ ID NO: 8) were synthesized. These two primers were used to perform a PCR reaction, using the genomic DNA of Streptomyces sp.720 strain as a template. An XGP-720 strain was obtained after the same procedures performed as above. It was confirmed that the resulting PCR fragment was identical to SEQ ID NO: 4 upon determining its base sequence in a DNA sequencer analysis. The primers of SEQ ID NOS: 5 to 8 are presented in Table 3.

**[Table 3]**

| Primers of SEQ ID NO: 5 to 8 | | |
|---|---|---|
| SEQ ID NO: 5 | XGP-710 primer (1) | ggaattccatatgaccgagctcgccgcggt |
| SEQ ID NO: 6 | XGP-710 primer (2) | cccaagcttctacgccccccacccggcctg |
| SEQ ID NO: 7 | XGP-720 primer (1) | ggaattccatatgaccgagctcgccgcggt |
| SEQ ID NO: 8 | XGP-720 primer (2) | cccaagctttcacgctccccagcccgcctg |
| Restriction enzymes: NdeI and HindIII | | |

Host cells with the expression system above were cultured to obtain a crude enzyme solution.

Subsequently, the XGP-710 and XGP-720 strains were each inoculated in a TSB medium (50 mL) in a 500-mL baffled flask, and rotary cultured under 28°C, 160 rpm conditions. On day 3 of culture, each culture was collected into a 50-mL centrifuge tube. The cells were centrifuged at 9000 rpm for 10 minutes with a HITACHI High-Speed Micro Centrifuge (Model CF15RXII), and the supernatant was discarded. The bacteria were washed once with distilled water, and recentrifuged. After discarding the supernatant, the bacteria were each suspended in 30 ml of a 40 mM tris-maleate buffer (pH 7.0), and sonicated twice, each for 20 seconds, with an Astrason Ultrasonic Cell Disrupter (W385; HEAT SYSTEM) operated in a 50% duty cycle at an output control level of 5. The disrupted solution was centrifuged at 9000 rpm for 10 minutes, and the supernatant was collected. The liquid was then membrane filtered with a Minisart (1.2 µm; Sartorius) to obtain a crude enzyme solution (an XGP-710 crude enzyme solution, and an XGP-720 crude enzyme solution).

### Example 8

D-Allose can be produced by using a chemical or a biological method. Chemically, D-allose can be produced from D-ribose (Non Patent Literature 5), or through reduction of 1,2:5,6-di-O-isopropylidene-α-D-ribohexofuranose-3-ulose (Non Patent Literature 6). Enzymatically, D-allose can be produced from D-psicose with L-riboseisomerase (L-RhI) from Pseudomonas stutzerii LL172 (Non Patent Literature 7).

In the present invention, the characteristics of the isomerase obtained from Streptomyces were examined.

### (1) Enzyme Reactivity

First, the enzyme was examined for its reactivity to different substrates.

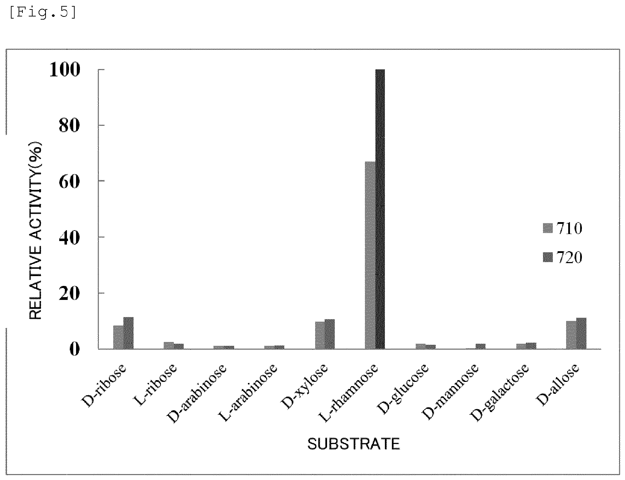

Aldoses were used as substrates. Specifically, L-rhamnose, D-ribose, D-arabinose, L-arabinose, D-xylose, D-glucose, and D-allose were reacted under the foregoing enzyme activity reaction conditions, and the resulting reaction mixtures were each measured for enzyme activity by using the cysteine carbazole method. The enzyme showed the highest substrate activity to L-rhamnose, and higher to D-xylose, D-ribose, D-allose, D-glucose, and L-arabinose (FIG. 5).

### (2) Effect of Metals on Enzyme Activity

Subsequently, in order to examine the effect of metal ions on isomerase activity, the enzyme was partially dialyzed to measure the enzyme activity. For dialysis, the crude enzyme solution was applied to a cellulose film, and immersed in a glycine-NaOH buffer containing 20 mM EDTA (pH 9.0), and the buffer was slowly stirred over a time period of 16 hours to eliminate the effect of other metal ions. The enzyme activity of the resulting apoenzyme was then measured by using the cysteine carbazole method after performing reaction in the presence of various divalent ions (under the reaction conditions presented in Table 4).

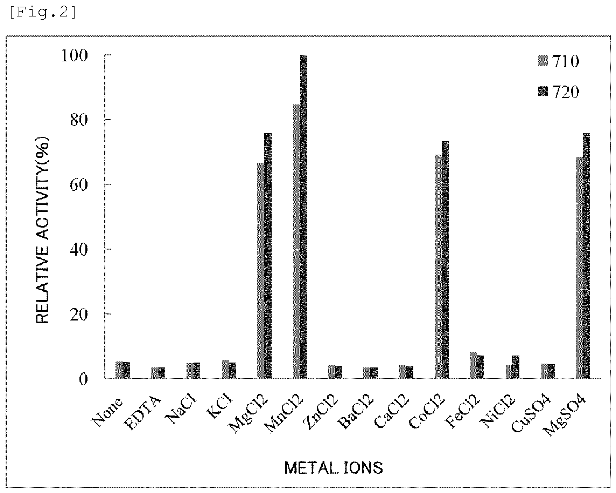

It was found that MnCl₂ greatly increased the enzyme activity. On the other hand, MgSO₄, MgCl₂, and CoCl₂ only slightly increased the activity, though the enzyme activity was shown to be metal dependent. CaCl₂, BaCl₂, ZnCl₂, and CuSO₄ inhibited the activity (FIG. 2).

It is reported that L-RhI expressed in E. coli requires Mn²⁺ or Zn²⁺ to exhibit its enzyme activity (Non Patent Literature 8).

**[Table 4]**

| Reaction Conditions for Checking the Effect of Metal Ions | |
|---|---|
| Reaction Conditions | |
| Buffer (Gly-NaOH) | 350 µL |
| Substrate (50 mM L-rhamnose) | 50 µL |
| Dialysis Enzyme (0.10 mg/50 µL) | 50 µL |
| Coenzyme (10 mM of each metal salt) | 50 µL |
| Reaction temperature | 60°C |
| Reaction time | 10 min |

### (3) Effect of Temperature on Enzyme Activity (Optimum Temperature)

The effect of temperature on enzyme activity was then examined.

Enzyme reactions were performed under varying reaction temperatures 10, 20, 30, 40, 50, 60, 70, 80, and 90°C under the conditions of Table 5, and the enzyme activity was determined through measurements using the cysteine carbazole method.

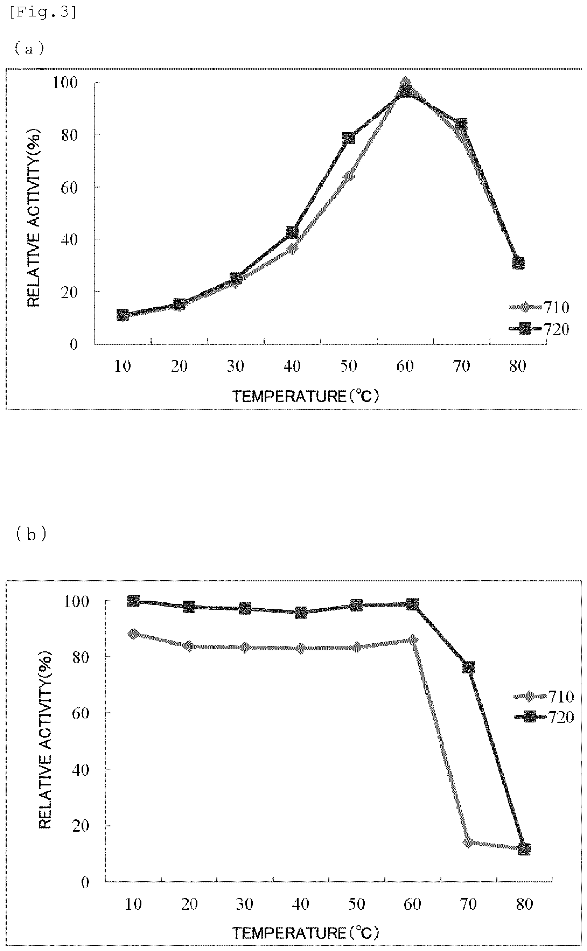

The optimum temperature for the enzyme activity was found to be 60°C (FIG. 3a).

**[Table 5]**

| Reaction Conditions for Checking the Effect of Temperature | |
|---|---|
| Reaction Conditions | |
| Buffer (Gly-NaOH) | 350 µL |
| Substrate (50 mM L-rhamnose) | 50 µL |
| Enzyme solution (0.10 mg/50 µL) | 50 µL |
| Coenzyme (10 mM MnCl₂) | 50 µL |
| Reaction temperature | Varying temperatures |
| Reaction time | 10 min |

### (4) Heat Resistance of Enzyme Activity

The heat resistance of the enzyme activity was then examined.

Each enzyme solution was maintained in a glycine-NaOH buffer (pH 9.0) for 1 hour under different temperature conditions (10, 20, 30, 40, 50, 60, 70, 80°C), and the remaining enzyme activity was measured by using the cysteine carbazole method after the enzyme reaction performed under the foregoing conditions.

About 80 to 90% enzyme activity remained even when maintained for 1 hour at 60°C (FIG. 3b) .

### (5) Effect of pH on Enzyme Activity (Optimum pH)

The effect of pH on enzyme activity was then examined.

Specifically, the enzyme solution was maintained in different pH buffers (50 mM citrate buffer (pH 3.0-6.0), 50 mM sodium phosphate buffer (pH 6.0-8.0), 50 mM tris-hydrochloride buffer (pH 7.0-9.0), and 50 mM glycine-sodium hydroxide buffer (pH 9.0-11.0)) at 4°C for 24 hours. Enzyme reactions were performed under the reaction conditions of Table 6, and the remaining enzyme activity was measured by using the cysteine carbazole method. The D-allose to D-psicose isomerization reaction conditions are presented in the table below.

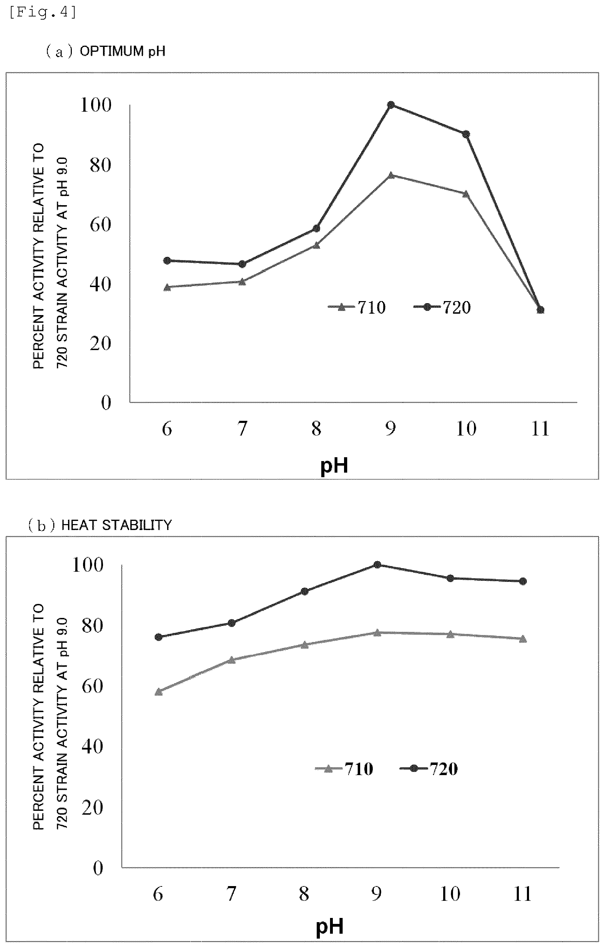

It was found that the optimum pH for the enzyme was 9.0 (FIG. 4a), and the enzyme activity was more stable in the pH 9.0 glycine-sodium hydroxide buffer (FIG. 4b).

**[Table 6]**

| Reaction Conditions for Checking the Effect of pH | |
|---|---|
| Reaction Conditions | |
| Buffer (Gly-NaOH) | 350 µL |
| Substrate (50 mM L-rhamnose) | 50 µL |
| Enzyme solution after left for 24 hours at 4°C | 50 µL |
| Coenzyme (10 mM MnCl₂) | 50 µL |
| Reaction temperature | 60°C |
| Reaction time | 10 min |

### (6) Percentage Yield of Rare Sugar at the Equilibrium Point of Enzyme Catalyzed Reaction

The enzyme was then reacted with each of the aldose substrates L-rhamnose, D-ribose, D-arabinose, L-arabinose, D-xylose, D-glucose, and D-allose under the reaction conditions of Table 7 (the reaction time was 6 to 48 hours), and the sugar composition of the reaction liquid at the equilibrium point was examined by HPLC.

**[Table 7]**

| Reaction Conditions for Different Aldose Substrates until Equilibrium Point | |
|---|---|
| Reaction Conditions | |
| Buffer (Gly-NaOH) pH: 9.0 | 200 µL |
| Substrate (0.2 M of each aldose) | 375 µL |
| Enzyme solution (0.10 mg/50 µL) | 100 µL |
| Coenzyme (0.01 M MnCl₂) | 75 µL |
| Temperature | 55°C |
| Reaction time | 6 to 48 hours |

Table 8 presents the percentage yield of ketose and aldose from each aldose acted upon by the enzyme (XGP-720 enzyme solution).

**[Table 8]**

| Substrate | Pr roduct^{a} | | Percentage Yield^{b} (%) | Time (hr) |
|---|---|---|---|---|
| Aldose | Ketose | Aldose | | |
| L-Rhamnose | L-Rhamnulose | None | 60:40:0 | 6 |
| D-Ribose | D-Ribulose | D-Arabinose | 65:22:13 | 24 |
| D-Arabinose | D-Ribulose | D-Ribose | 91:2:7 | 24 |
| L-Arabinose | L-Ribulose | None | 99:1:0 | 48 |
| D-Xylose | D-Xylulose | D-Lyxose | 82:16:2 | 24 |
| D-Glucose | D-Fructose | None | 83:17:0 | 48 |
| D-allose | D-Psicose | D-Altrose | 33:66:1 | 24 |

| | | | | |
|---|---|---|---|---|
| ^{a}Ketose was produced first in all reactions ^{b}Percentage yield represents aldose (remaining substrate):ketose (product):aldose (product) at the equilibrium point. | | | | |

### (7) Isomerase Reaction between D-Psicose and D-Allose

The D-psicose to D-allose isomerase reaction was then examined.

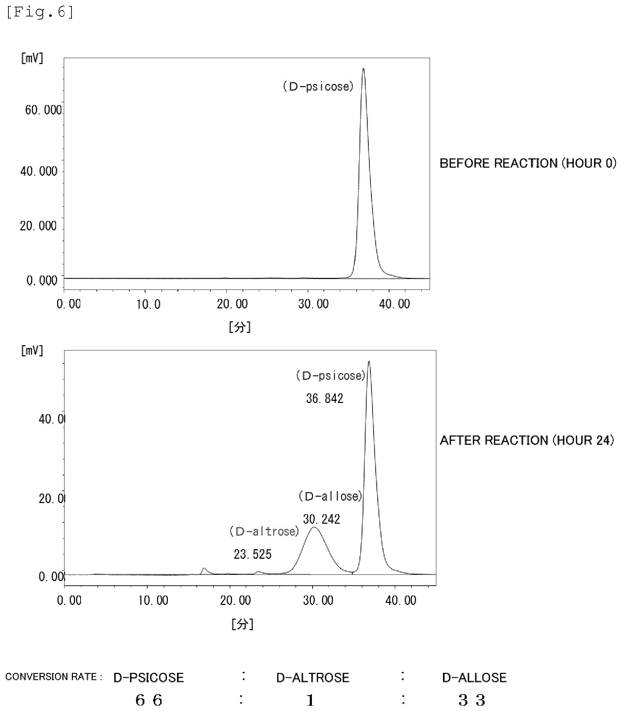

Enzyme reactions were performed under the reaction conditions of Table 9, and the sugar composition was measured by HPLC. FIG. 6 represents the result of the HPLC analysis before (hour 0, D-psicose) and after (a D-psicose, D-altrose, and D-allose mixture after 24-hour reaction) the reaction.

It was confirmed that the enzyme can generate D-allose from D-psicose in the D-psicose:D-allose:D-altrose ratio of 66:33:1 in the enzyme reaction performed by using D-psicose as a substrate. This was in conformity with the conversion rate given above for each aldose substrate.

**[Table 9]**

| Reaction Con ditions | |
|---|---|
| Buffer (Gly-NaOH) pH: 9.0 | 200 µL |
| Substrate (0.2 M D-psicose) | 375 µL |
| Enzyme solution (0.10 mg/50 µL) | 100 µL |
| Coenzyme (0.01 M MnCl₂) | 75 µL |
| Temperature | 55°C |
| Reaction time | 24 hours |

### [Conclusion]

The both enzymes had high affinity to L-rhamnose, though the activity was slightly higher in the XGP-720 enzyme than in the XGP-710 enzyme. These enzymes have an optimum temperature and stability in the vicinity of 60°C, which should be effective at preventing microorganism contamination. The enzymes showed the highest activity in a pH 9.0 glycine-sodium hydroxide buffer in the presence of MnCl₂ metal ions. These results clearly suggest that the enzymes of the Streptomyces bacterial strains identified herein are effective for the production of D-allose from D-psicose.

### Industrial Applicability

The enzyme of the present invention is capable of producing D-allose through isomerization of D-psicose, and the microorganisms that produce this enzyme are strains of genus Streptomyces. Streptomyces microorganisms have been used in food production, and are considered safe. The most notable feature of using these enzyme-producing Streptomyces strains in food industry is the safety of the bacteria. Enzymes from microorganisms of genera Agrobacterium, Rhizobium, and Pseudomonas are known to produce D-allose and other rare sugars. However, these are reported as being opportunistic or causative of plant cell infection, and require a large labor force to check safety. The present invention enabling use of highly safe bacteria or bacterial culture is a large technological advancement. The present invention providing the enzyme capable of producing D-allose through isomerization of D-psicose, and the established method of production of such enzymes is thus considered to be industrially highly significant not only in sugar production but in related food, cosmetic, and drug industries.

## Claims

1. A method for producing D-allose,
the method comprising isomerizing D-psicose to D-allose by reacting the D-psicose with a protein comprising the amino acid sequence of SEQ ID NO: 1 or SEQ ID NO: 2.

2. The method for producing D-allose according to claim 1, wherein the D-psicose is produced by epimerizing D-fructose.

3. The method for producing D-allose according to claim 1, wherein the D-psicose is produced by directing D-glucose to D-fructose through isomerization, and epimerizing the D-fructose.

4. The method for producing D-allose according to claim 1, wherein the D-psicose is produced by obtaining D-glucose from an unused resource, directing the D-glucose into D-fructose through isomerization, and epimerizing the D-fructose.

5. The method for producing D-allose according to any one of claims 1 to 4, wherein the target product D-allose is a mixture of D-psicose and D-allose.

6. The method according to any one of claims 1 to 5, wherein said protein comprises the amino acid sequence of SEQ ID NO: 1 or SEQ ID NO: 2 is produced by a host cell transformed with a recombinant vector which comprises a DNA having the nucleotide sequence of SEQ ID NO: 3 or SEQ ID NO: 4.

## Patentansprüche

1. Verfahren zur Herstellung von D-Allose,
wobei das Verfahren die Isomerisierung von D-Psicose zu D-Allose durch Reaktion der D-Psicose mit einem Protein umfasst, das die Aminosäuresequenz der SEQ ID NO: 1 oder SEQ ID NO: 2 umfasst.

2. Verfahren zur Herstellung von D-Allose nach Anspruch 1, wobei die D-Psicose durch Epimerisierung von D-Fructose hergestellt wird.

3. Verfahren zur Herstellung von D-Allose nach Anspruch 1, wobei die D-Psicose durch Überführen von D-Glucose zu D-Fructose durch Isomerisierung und Epimerisierung der D-Fructose hergestellt wird.

4. Verfahren zur Herstellung von D-Allose nach Anspruch 1, wobei die D-Psicose durch Erhalten von D-Glucose aus einer ungenutzten Quelle, Überführen der D-Glucose zu D-Fructose durch Isomerisierung und Epimerisierung der D-Fructose hergestellt wird.

5. Verfahren zur Herstellung von D-Allose nach einem der Ansprüche 1 bis 4, wobei das Zielprodukt D-Allose eine Mischung aus D-Psicose und D-Allose ist.

6. Verfahren nach einem der Ansprüche 1 bis 5, wobei das Protein, das die Aminosäuresequenz der SEQ ID NO: 1 oder SEQ ID NO: 2 umfasst, von einer Wirtszelle produziert wird, die mit einem rekombinanten Vektor transformiert ist, der eine DNA mit der Nukleotidsequenz der SEQ ID NO: 3 oder SEQ ID NO: 4 umfasst.

## Revendications

1. Procédé pour produire du D-allose, le procédé comprenant l'isomérisation de D-psicose en D-allose par réaction du D-psicose avec une protéine comprenant la séquence d'acides aminés de la SEQ ID NO : 1 ou de la SEQ ID NO : 2.

2. Procédé pour produire du D-allose selon la revendication 1, dans lequel le D-psicose est produit par épimérisation de D-fructose.

3. Procédé pour produire du D-allose selon la revendication 1, dans lequel le D-psicose est produit par transformation de D-glucose en D-fructose par isomérisation, et épimérisation du D-fructose.

4. Procédé pour produire du D-allose selon la revendication 1, dans lequel le D-psicose est produit par obtention de D-glucose à partir d'une ressource inutilisée, transformation du D-glucose en D-fructose par isomérisation, et épimérisation du D-fructose.

5. Procédé pour produire du D-allose selon l'une quelconque des revendications 1 à 4, dans lequel le produit cible D-allose est un mélange de D-psicose et de D-allose.

6. Procédé selon l'une quelconque des revendications 1 à 5, dans lequel ladite protéine comprenant la séquence d'acides aminés de la SEQ ID NO : 1 ou de la SEQ ID NO : 2 est produite par une cellule hôte transformée par un vecteur recombiné qui comprend l'ADN ayant la séquence de nucléotides de la SEQ ID NO : 3 ou de la SEQ ID NO : 4.


### SEQUENCE LISTING

<110> MATSUTANI CHEMICAL INDUSTRY CO., LTD. NAGASE CO.,LDT. NAGASE CHEMTEX CORPORATION\201@
<120> A PROCESS FOR THE PRODUCTION OF D-ALLOSE\201@
<130> PCT13053MC
<160> 8
<170> PatentIn version 3.1
<210> 1
   <211> 386
   <212> PRT
   <213> Streptomyces 710
<400> 1
   Met Thr Glu Leu Ala Ala Val Lys Ala Ala Leu Lys Thr Gln Ala Val
   1               5                   10                  15
   Glu Thr Pro Ser Trp Ala Tyr Gly Asn Ser Gly Thr Arg Phe Lys Val
               20                  25                  30
   Phe Ala Gln Pro Gly Val Pro Arg Asp Pro Phe Glu Lys Leu Asp Asp
           35                  40                  45
   Ala Ala Lys Val His Glu Phe Thr Gly Ala Ala Pro Thr Val Ala Leu
       50                  55                  60
   His Ile Pro Trp Asp Arg Val Glu Asp Tyr Ala Ala Leu Ala Ala His
   65                  70                  75                  80
   Ala Glu Lys Arg Gly Val Arg Ile Gly Ala Ile Asn Ser Asn Thr Phe
                   85                  90                  95
   Gln Asp Asp Asp Tyr Arg Leu Gly Ser Ile Cys His Pro Asp Ala Ala
               100                 105                 110
   Val Arg Arg Lys Ala Val Asp His Leu Leu Glu Cys Val Asp Ile Met
           115                 120                 125
   Asp Ala Thr Gly Ser Arg Asp Leu Lys Leu Trp Phe Ala Asp Gly Thr
       130                 135                 140
   Asn Tyr Pro Gly Gln Asp Asp Ile Arg Ser Arg Gln Asp Arg Leu Ala
   145                 150                 155                 160
   Glu Gly Leu Ala Glu Val Tyr Glu Arg Leu Gly Glu Gly Gln Arg Met
                   165                 170                 175
   Leu Leu Glu Tyr Lys Leu Phe Glu Pro Ala Phe Tyr Thr Thr Asp Val
               180                 185                 190
   Pro Asp Trp Gly Thr Ala Tyr Ala His Cys Leu Lys Leu Gly Glu Lys
           195                 200                 205
   Ala Gln Val Val Val Asp Thr Gly His His Ala Pro Gly Thr Asn Ile
       210                 215                 220
   Glu Phe Ile Val Ala Thr Leu Leu Arg Glu Gly Lys Leu Gly Gly Phe
   225                 230                 235                 240
   Asp Phe Asn Ser Arg Phe Tyr Ala Asp Asp Asp Leu Met Val Gly Ala
                   245                 250                 255
   Ala Asp Pro Phe Gln Leu Phe Arg Ile Met Tyr Glu Val Val Arg Gly
               260                 265                 270
   Gly Gly Phe Thr Ser Asp Val Ala Phe Met Leu Asp Gln Cys His Asn
           275                 280                 285
   Ile Glu Ala Lys Ile Pro Ala Ile Ile Arg Ser Val Met Asn Val Gln
       290                 295                 300
   Glu Ala Thr Ala Lys Ala Leu Leu Val Asp Gly Thr Ala Leu Ala Glu
   305                 310                 315                 320
   Ala Gln Ala Ala Gly Asp Val Leu Glu Ala Asn Ala Val Leu Met Asp
                   325                 330                 335
   Ala Tyr Asn Thr Asp Val Arg Pro Leu Leu Arg Glu Val Arg Glu Glu
               340                 345                 350
   Ser Gly Leu Asp Pro Glu Pro Met Lys Ala Tyr Arg Ser Cys Gly Trp
           355                 360                 365
   Ala Glu Lys Val Val Ala Glu Arg Ile Gly Gly Gln Gln Ala Gly Trp
       370                 375                 380
   Gly Ala
   385
<210> 2
   <211> 386
   <212> PRT
   <213> Streptomyces 720
<400> 2
   Met Thr Glu Leu Ala Ala Val Lys Ala Ala Leu Lys Thr Gln Ala Val
   1               5                   10                  15
   Glu Thr Pro Ser Trp Ala Tyr Gly Asn Ser Gly Thr Arg Phe Lys Val
               20                  25                  30
   Phe Ala Gln Pro Gly Val Pro Arg Thr Pro Gln Glu Lys Leu Asp Asp
           35                  40                  45
   Ala Ala Gln Val His Ala Tyr Thr Gly Ala Ala Pro Thr Val Ala Leu
       50                  55                  60
   His Ile Pro Trp Asp Arg Val Glu Asp Tyr Ala Lys Leu Ala Glu Tyr
   65                  70                  75                  80
   Ala Glu Glu Arg Gly Leu Lys Leu Gly Thr Ile Asn Ser Asn Thr Phe
                   85                  90                  95
   Gln Asp Asp Ala Tyr Lys Leu Gly Ser Ile Cys His Pro Glu Ala Ala
               100                 105                 110
   Val Arg Arg Lys Ala Leu Asp His Leu Leu Glu Cys Val Asp Ile Met
           115                 120                 125
   Asp Ala Thr Gly Ser Lys Asp Leu Lys Leu Trp Phe Ala Asp Gly Thr
       130                 135                 140
   Asn Tyr Pro Gly Gln Asp Asp Val Arg Glu Arg Gln Asp Arg Leu Thr
   145                 150                 155                 160
   Glu Gly Leu Ala Thr Val Tyr Glu Arg Leu Gly Asp Asp Gln Arg Met
                   165                 170                 175
   Leu Leu Glu Tyr Lys Phe Phe Glu Pro Ala Phe Tyr Thr Thr Asp Val
               180                 185                 190
   Pro Asp Trp Gly Thr Ala Tyr Ala His Cys Leu Lys Leu Gly Asp Arg
           195                 200                 205
   Ala Gln Val Val Val Asp Thr Gly His His Ala Pro Gly Thr Asn Ile
       210                 215                 220
   Glu Phe Ile Val Ala Thr Leu Leu Arg Glu Gly Lys Leu Gly Gly Phe
   225                 230                 235                 240
   Asp Phe Asn Ser Arg Phe Tyr Ala Asp Asp Asp Leu Met Val Gly Ala
                   245                 250                 255
   Ala Asp Pro Phe Gln Leu Phe Arg Ile Met Tyr Glu Val Ile Arg Gly
               260                 265                 270
   Gly Gly Leu Thr Ser Asp Val Ala Phe Met Leu Asp Gln Cys His Asn
           275                 280                 285
   Ile Glu Ala Lys Ile Pro Ala Ile Ile Arg Ser Val Met Asn Val Gln
       290                 295                 300
   Glu Ala Thr Ala Lys Ala Leu Leu Val Asp Arg Glu Ala Leu Arg Ser
   305                 310                 315                 320
   Ala Gln Arg Ser Gly Asp Val Leu Glu Ala Asn Ala Val Leu Met Asp
                   325                 330                 335
   Ala Tyr Asn Thr Asp Val Arg Pro Leu Leu Ala Glu Leu Arg Glu Glu
               340                 345                 350
   Thr Gly Leu Asp Pro Asp Pro Val Ala Ala Tyr Arg Arg Ser Gly Trp
           355                 360                 365
   Ala Ser Lys Ile Ala Glu Glu Arg Val Gly Gly Gln Gln Ala Gly Trp
       370                 375                 380
   Gly Ala
   385
<210> 3
   <211> 1161
   <212> DNA
   <213> Streptomyces 710
<400> 3
   gtgaccgagc tcgccgcggt gaaagccgcg ctcaagaccc aggccgtcga gacgccgtcg 60
   tgggcgtacg gcaactcggg cacccgcttc aaggtgttcg cccagcccgg tgtcccgcgc 120
   gaccccttcg agaaactgga cgacgcggcg aaggtgcacg agttcaccgg cgcggccccg 180
   accgtcgccc tgcacatccc gtgggaccgg gtcgaggact acgccgcgct ggcggcgcac 240
   gccgagaagc ggggcgtgcg gatcggcgcg atcaactcca acaccttcca ggacgacgac 300
   tacaggctgg gcagcatctg ccaccccgac gcggcggtgc gccggaaggc cgtcgaccac 360
   ctgctggagt gcgtcgacat catggacgcc accggctcgc gcgacctgaa gctgtggttc 420
   gccgacggca cgaactaccc gggacaggac gacatccggt cccggcagga ccggctggcc 480
   gagggcctgg ccgaggtgta cgagcggctc ggcgaggggc agcggatgct cctggagtac 540
   aagctcttcg agccggcgtt ctacacgacg gacgtgccgg actggggcac ggcgtacgcg 600
   cactgtctca agctgggcga gaaggcccag gtcgtggtcg acacgggaca ccacgcgccg 660
   ggcacgaaca tcgagttcat cgtggcgacg ctgctgcggg aggggaagct cggcgggttc 720
   gacttcaact cgcggttcta cgccgacgac gacctgatgg tgggtgccgc cgacccgttc 780
   cagctgttcc ggatcatgta cgaggtggtg cgcgggggcg ggttcacctc cgacgtggcg 840
   ttcatgctcg accagtgcca caacatcgag gcgaagatcc cggcgatcat ccggtcggtg 900
   atgaacgtgc aggaggcgac ggcgaaggcg ctgctggtcg acggcaccgc cctggccgag 960
   gcgcaggccg cgggggatgt gctggaggcg aacgcggtgc tgatggacgc gtacaacacg 1020
   gacgtgcggc cgctgctccg ggaagtgcgg gaggagtcgg ggctggaccc cgaacccatg 1080
   aaggcgtacc gctcctgcgg gtgggcggag aaggtcgttg ccgagcggat cggtgggcag 1140
   caggccgggt ggggggcgta g 1161
<210> 4
   <211> 1161
   <212> DNA
   <213> Streptomyces 720
<400> 4
   gtgaccgagc tcgccgcggt gaaggccgcc ctcaagaccc aggccgtaga gacgccgtca      60
   tgggcgtacg ggaactcggg gacccgcttc aaggtgttcg cgcagccggg agtcccccgc     120
   acgccccagg agaagctcga cgacgccgcg caggtgcacg cgtacacggg tgccgcgccg     180
   accgtggcac tgcacatccc ctgggaccgg gtcgaggact acgcgaagct cgccgagtac     240
   gccgaggagc gcgggctgaa gctggggacg atcaactcca acaccttcca ggacgacgcg     300
   tacaagctcg gcagcatctg ccaccccgag gcggccgtgc gccgcaaagc ccttgatcac     360
   ttgctcgagt gcgtcgacat catggacgcg accggttcga aggacctgaa gctgtggttc     420
   gcggacggca ccaactatcc cggccaggac gatgtccgtg agcgccagga ccgcctcacc     480
   gaagggctgg ccacggtgta cgagcggctc ggtgacgacc agcggatgct gctcgagtac     540
   aagttcttcg agcccgcctt ctacacgacg gacgtgccgg actggggcac ggcgtacgcg     600
   cactgcctca agctcggtga cagggcgcag gtcgtggtcg acaccgggca tcacgcaccc     660
   ggcaccaaca tcgagttcat cgtggccaca ctgctgcgcg aggggaagct cggcggcttc    720
   gacttcaact cccgcttcta cgccgacgac gacctgatgg tgggcgccgc cgaccccttc    780
   cagctgttcc ggatcatgta cgaggtgatc cgcggcggcg gcctcacgtc cgacgtggcc     840
   ttcatgctcg accagtgcca caacatcgag gcgaagatcc cggcgatcat ccgctcggtg     900
   atgaacgtcc aggaggcgac ggcgaaggcg ctgctcgtgg atcgtgaggc gctgcggtcg     960
   gcgcagcggt ccggggacgt gctggaggcg aacgcggtcc tcatggacgc gtacaacacc    1020
   gatgtacggc cgctgctcgc cgagctgcgc gaggagacgg ggctcgaccc ggacccggtc    1080
   gccgcgtacc ggcggtccgg gtgggcgtcg aagatcgccg aggagcgggt gggtggtcag    1140
   caggcgggct ggggagcgtg a                                              1161
<210> 5
   <211> 30
   <212> DNA
   <213> Artificial Sequence
   <223> primer
<400> 5
   ggaattccat atgaccgagc tcgccgcggt 30
<210> 6
   <211> 30
   <212> DNA
   <213> Artificial Sequence
   <223> primer
<400> 6
   cccaagcttc tacgcccccc acccggcctg 30
<210> 7
   <211> 30
   <212> DNA
   <213> Artificial Sequence
   <223> primer
<400> 7
   ggaattccat atgaccgagc tcgccgcggt 30
<210> 8
   <211> 30
   <212> DNA
   <213> Artificial Sequence
   <223> primer
<400> 8
   cccaagcttt cacgctcccc agcccgcctg 30